# EUROPEAN PATENT SPECIFICATION

(11) **EP 1 784 227 B1**
(45) Date of publication and mention of the grant of the patent: **05.10.2011**
(21) Application number: 05771198.8
(22) Date of filing: 28.07.2005
(51) Int. Cl.: G01R 33/485, G01R 33/56, A61B 5/055, A61K 49/06

(54) **MR IMAGING METHOD FOR THE DISCRIMINATION BETWEEN HEALTHY AND TUMOUR TISSUE**
MR-DARSTELLUNGSVERFAHREN ZUR DISKRIMINIERUNG ZWISCHEN GESUNDEM UND TUMORGEWEBE
PROCEDE D'IMAGERIE DE TUMEURS

(30) Priority: 30.07.2004 NO 20043226
(43) Date of publication of application: 16.05.2007
(73) Proprietor: GE HEALTHCARE AS, 0401 Oslo (NO)
(72) Inventor: THANING, Mikkel, N-0401 Oslo (NO); ZANDT, René in't, SE-247 36 Södra Sandby (SE)
(74) Representative: Wulff, Marianne Weiby
(86) International application number: PCT/NO2005/000282
(87) International publication number: WO 2006/011810

(56) References cited:
- EP-A- 1 574 874
- US-B1- 6 466 814
- GOULD P.: "C-13 MR tracers show potential for functional diagnostics" DIAGNOSTIC IMAGING, [Online] June 2004 (2004-06), XP002363324 Retrieved from the Internet: URL:http://www.diagnosticimaging.com/molec ularimagingoutlook/2004jun/04.jhtml> [retrieved on 2006-01-17]
- ALBERS M.J. ET AL: "A 13C HR-MAS Technique for Studying the Cellular Bioenergetics Associated with Prostate Cancer" PROCEEDINGS OF THE INTERNATIONAL SOCIETY FOR MAGNETIC RESONANCE IN MEDICINE, 13TH MEETING PROCEEDINGS, 7 May 2005 (2005-05-07), page 2131, XP002363326 Miami Beach, Florida, USA
- GOLMAN K ET AL: "Molecular imaging using hyperpolarized 13C." THE BRITISH JOURNAL OF RADIOLOGY. 2003, vol. 76 Spec No 2, 2003, pages S118-S127, XP002363325 ISSN: 0007-1285
- WOLBER J ET AL: "Generating highly polarized nuclear spins in solution using dynamic nuclear polarization" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 526, no. 1-2, 21 June 2004 (2004-06-21), pages 173-181, XP004830097 ISSN: 0168-9002
- GILLIES R.J.: "NMR in Physiology and Biomedicine" 1994, ACADEMIC PRESS , USA , XP002363328 *Chapter 19: Bhujwalla Z.M. et al, "MR Studies of Tumors: Relationship between Blood Flow, Metabolism and Physiology" *

## Description

The invention relates to a method tumour imaging using hyperpolarised ¹³C-pyruvate as MR imaging agent, the method allowing for the discrimination between tumour tissue and healthy tissue.

Magnetic resonance (MR) imaging (MRI) is a imaging technique that has become particularly attractive to physicians as it allows for obtaining images of a patients body or parts thereof in a non-invasive way and without exposing the patient and the medical personnel to potentially harmful radiation such as X-ray. Because of its high quality images, MRI is the favourable imaging technique of soft tissue and organs and it allows for the discrimination between normal and diseased tissue, for instance tumours and lesions.

MR tumour imaging may be carried out with or without MR contrast agents. On an MR image taken without contrast agent, tumours from about 1-2 centimetres in size and larger will show up fairly clearly. However, contrast-enhanced MRI enables much smaller tissue changes, i.e. much smaller tumours to be detected which makes contrast-enhanced MR imaging a powerful tool for early stage tumour detection and detection of metastases.

Several types of contrast agents have been used in MR tumour imaging. Watersoluble paramagnetic metal chelates, for instance gadolinium chelates like Omniscan^{™} (Amersham Health) are widely used MR contrast agents. Because of their low molecular weight they rapidly distribute into the extracellular space (i.e. the blood and the interstitium) if administered into the vasculature. They are also cleared relatively rapidly from the body. Gadolinium chelates have been found to be especially useful in increasing the detection rate of metastases, small tumours, and improving tumour classification, the latter by allowing the differentiation of vital tumour tissue (well perfused and/or impaired blood-brain-barrier) from central necrosis and from surrounding oedema or macroscopically uninvolved tissue (see for instance C. Claussen et al., Neuroradiology 1985; 27: 164-171).

Blood pool MR contrast agents on the other hand, for instance superparamagnetic iron oxide particles, are retained within the vasculature for a prolonged time. They have proven to be extremely useful to enhance contrast in the liver but also to detect capillary permeability abnormalities, e.g. "leaky" capillary walls in tumours for example as a result of angiogenesis.

Despite the undisputed excellent properties of the aforementioned contrast agents their use is not without any risks. Although paramagnetic metal chelate complexes have usually high stability constants, it is possible that toxic metal ions are released in the body after administration. Further, these type of contrast agents show poor specificity.

WO-A-99/35508 discloses a method of MR investigation of a patient using a hyperpolarised solution of a high T₁ agent as MR imaging agent. The term "hyperpolarisation" means enhancing the nuclear polarisation of NMR active nuclei present in the high T₁ agent, i.e. nuclei with non-zero nuclear spin, preferably ¹³C- or ¹⁵ N-nuclei. Upon enhancing the nuclear polarisation of NMR active nuclei, the population difference between excited and ground nuclear spin states of these nuclei are significantly increased and thereby the MR signal intensity is amplified by a factor of hundred and more. When using a hyperpolarised ¹³C- and/or ¹⁵N-enriched high T₁ agent, there will be essentially no interference from background signals as the natural abundance of ¹³C and/or ¹⁵N is negligible and thus the image contrast will be advantageously high. A variety of possible high T₁ agents suitable for hyperpolarisation and subsequent use as MR imaging agents are disclosed including but not limited to non-endogenous and endogenous compounds like acetate, pyruvate, oxalate or gluconate, sugars like glucose or fructose, urea, amides, amino acids like glutamate, glycine, cysteine or aspartate, nucleotides, vitamins like ascorbic acid, penicillin derivates and sulfonamides. It is further stated that intermediates in normal metabolic cycles such as the citric acid cycle like fumaric acid and pyruvic acid are preferred imaging agents for the imaging of metabolic activity. The article Gould, P, Diagnostic Imaging, June 2004 discloses a method for the discrimination between healthy and tumour tissue, comprising acquiring direct 13C-MR images of 13C-pyruvate and its 13C - containing metabolites alanine and lactate from a subject pre-administered with a composition comprising hyperpolarised 13C-pyruvate.

It has to be stressed that the signal of a hyperpolarised imaging agent decays due to relaxation and - upon administration to the patient's body - dilution. Hence the T₁ value of the imaging agents in biological fluids (e.g. blood) must be sufficiently high to enable the agent to be distributed to the target site in the patient's body in a highly hyperpolarised state.

We have now surprisingly found a method for MR tumour imaging which allows the discrimination between tumour tissue and healthy tissue in which hyperpolarised ¹³C-pyruvate is used as an imaging agent.

Thus, the present invention provides a method for the discrimination between healthy and tumour tissue, said method comprising
(a) acquiring a ¹³C-MR image of ¹³C-pyruvate, a ¹³C-MR image of its ¹³C-containing metabolite alanine and a ¹³C-MR image of its ¹³C-containing metabolite lactate from a subject pre-administered with a composition comprising hyperpolarised ¹³C-pyruvate,
(b) correcting the lactate image for the amount of pyruvate and/or alanine by multiplying the lactate image by the inverted pyruvate and/or alanine image, a high image signal within said corrected lactate image(s) being indicative of tumour tissue.

Hyperpolarisation of NMR active ¹³C-nuclei may be achieved by different methods (e.g. described in WO-A-99/35508), preferred methods are polarisation transfer from a noble gas, "brute force", spin refrigeration and DNP. To obtain hyperpolarised ¹³C-pyruvate, it is preferred to either polarise ¹³C-pyruvate directly or to polarise ¹³C-pyruvic acid and convert the polarised ¹³C-pyruvic acid to polarised ¹³C-pyruvate, e.g. by neutralisation with a base.

A preferred way for obtaining hyperpolarised ¹³C-pyruvate is the polarisation transfer from a hyperpolarised noble gas. Noble gases having non-zero nuclear spin can be hyperpolarised, i.e. have their polarisation enhanced over the equilibrium polarisation, e.g. by the use of circularly polarised light. A hyperpolarised noble gas, preferably He or Xe, or a mixture of such gases, may be used to effect hyperpolarisation of ¹³C-nuclei. The hyperpolarisation may also be achieved by using an isotopically enriched hyperpolarised noble gas, preferably ³He or ¹²⁹Xe. The hyperpolarised gas may be in the gas phase, it may be dissolved in a liquid/solvent, or the hyperpolarised gas itself may serve as a solvent. Alternatively, the gas may be condensed onto a cooled solid surface and used in this form, or allowed to sublime. Intimate mixing of the hyperpolarised gas with the compound to be polarised is preferred. Hence, if ¹³C-pyruvic acid is polarised, which is a liquid at room temperature, the hyperpolarised gas is preferably dissolved in a liquid/solvent or serves as a solvent. If ¹³C pyruvate is polarised, the hyperpolarised gas is preferably dissolved in a liquid/solvent, which also dissolves pyruvate.

Another preferred way for obtaining hyperpolarised ¹³C-pyruvate is that polarisation is imparted to NMR active nuclei by thermodynamic equilibration at a very low temperature and high field. Hyperpolarisation compared to the operating field and temperature of the NMR spectrometer is effected by use of a very high field and very low temperature (brute force). The magnetic field strength used should be as high as possible, suitably higher than 1 T, preferably higher than 5 T, more preferably 15 T or more and especially preferably 20 T or more. The temperature should be very low, e.g. 4.2 K or less, preferably 1.5 K or less, more preferably 1.0 K or less, especially preferably 100 mK or less.

Another preferred way for obtaining hyperpolarised ¹³C-pyruvate is the spin refrigeration method. This method covers spin polarisation of a solid compound or system by spin refrigeration polarisation. The system is doped with or intimately mixed with suitable paramagnetic materials such as Ni²⁺, lanthanide or actinide ions in crystal form with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The pre-requisite for this method is that the paramagnetic species has a highly anisotropic g-factor. As a result of the sample rotation, the electron paramagnetic resonance will be brought in contact with the nuclear spins, leading to a decrease in the nuclear spin temperature. Sample rotation is carried out until the nuclear spin polarisation has reached a new equilibrium.

In a more preferred embodiment, DNP (dynamic nuclear polarisation) method is used to obtain hyperpolarised ¹³C-pyruvate. Polarisation is effected by a paramagnetic compound, the so-called paramagnetic agent or DNP agent. During the DNP process, energy, normally in the form of microwave radiation, is provided, which will initially excite the paramagnetic agent. Upon decay to the ground state, there is a transfer of polarisation from the unpaired electron of paramagnetic agent to the NMR active nuclei of the sample. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. The DNP technique is for example described in WO-A-98/58272 and in WO-A-01/96895. To obtain hyperpolarised ¹³C-pyruvate by the DNP method, either ¹³C-pyruvate and/or ¹³C-pyruvic acid can be used as the compound to be polarised.

Whether ¹³C-pyruvic acid and/or ¹³C-pyruvate is used depends mainly on the paramagnetic agent employed in the DNP process. If the paramagnetic agent is soluble in ¹³C-pyruvic acid, then ¹³C-pyruvic acid is preferably used and a liquid mixture, preferably a liquid solution is formed by the paramagnetic agent and ¹³C-pyruvic acid. If the paramagnetic agent is not soluble in ¹³C-pyruvic acid, then ¹³C-pyruvate and/or ¹³C-pyruvic acid and at least one co-solvent are used to form a liquid mixture, preferably a liquid solution. It has been found that the success of the DNP and thus the level of polarisation is dependent on the compound to be polarised and the paramagnetic agent being in intimate contact with each other. Hence the co-solvent is preferably a co-solvent or co-solvent mixture that dissolves both the paramagnetic agent and ¹³C-pyruvic acid and/or ¹³C-pyruvate. For ¹³C-pyruvate water is preferably used as a co-solvent.

Further, it has been found that higher polarisation levels are achieved by the DNP method when the sample mixture upon cooling/freezing forms a glass rather than a crystallized sample. Again, the formation of a glass allows a more intimate contact of the paramagnetic agent and the compound to be polarised. ¹³C-pyruvic acid is a good glass former and is therefore preferably used in the DNP process, whenever the paramagnetic agent is soluble in ¹³C-pyruvic acid. ¹³C-pyruvate is a salt and a liquid mixture of an aqueous solution of ¹³C-pyruvate and a paramagnetic agent will result in a crystallized sample upon freezing. To prevent this, it is preferred to add further co-solvents which are good glass formers like glycerol, propanediol or glycol.

Hence in one embodiment, ¹³C-pyruvate is dissolved in water to obtain an aqueous solution and a paramagnetic agent, glycerol and optionally a further co-solvent are added to form a liquid mixture. In a preferred embodiment, ¹³C-pyruvic acid, a paramagnetic agent and a co-solvent are combined to form a liquid mixture. In a most preferred embodiment, ¹³C-pyruvic acid and a paramagnetic agent are combined to form a liquid mixture. Intimate mixing of the compounds can be achieved by several means known in the art, such as stirring, vortexing or sonification.

The liquid mixture is then frozen before the DNP process is carried out. Cooling/freezing of the liquid mixture may be achieved by methods known in the art, e.g. by freezing the liquid mixture in liquid nitrogen or by simply placing it in the polarizer, where liquid helium will freeze the sample.

As described previously, dynamic nuclear polarisation (DNP) is a polarisation method where polarisation of the compound to be polarised is effected by a DNP agent, i.e. a paramagnetic agent/compound.

Many known paramagnetic compounds may be used as DNP agents, e.g. transition metals such as chromium (V) ions, organic free radicals such as nitroxide radicals, trityl radicals or magnetic particles. Such DNP agents are for instance described in WO-A-99/35508, WO-A-88/10419, WO-A-90/00904, WO-A-91/12024, WO-A-93/02711 or WO-A-96/39367.

In a preferred embodiment, a trityl radical of formula (I) 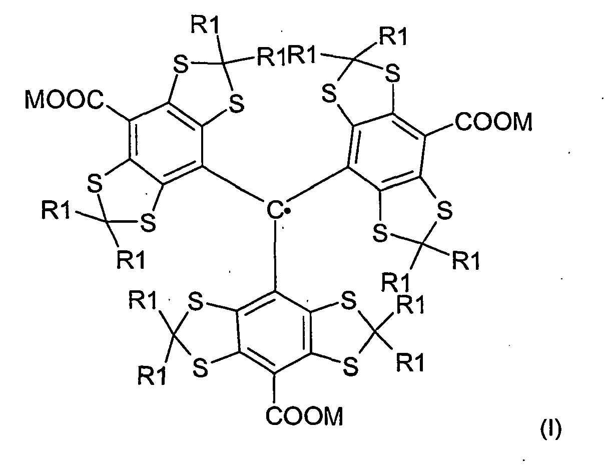 where
- M: represents hydrogen or one equivalent of a cation; and
- R1: which is the same or different represents a straight chain or branched optionally hydroxylated C₁-C₆-alkyl group or a group -(CH₂)ₙ-X-R2, wherein n is 1, 2 or 3; X is O or S and R2 is a straight chain or branched, optionally hydroxylated C₁-C₄-alkyl group.
is used as the paramagnetic agent to obtain ¹³C-pyruvate by the DNP method.

In a preferred embodiment, M represents hydrogen or one equivalent of a physiologically tolerable cation. The term "physiologically tolerable cation" denotes a cation that is tolerated by the human or non-human animal living body. Preferably, M represents hydrogen or an alkali cation, an ammonium ion or an organic amine ion, for instance meglumine. Most preferably, M represents hydrogen or sodium.

In a further preferred embodiment, R1 is the same or different, preferably the same and represents a straight chain or branched optionally hydroxylated C₁-C₄-alkyl group, most preferably methyl, ethyl, isopropyl, hydroxymethyl or hydroxyethyl.

In a further preferred embodiment, R1 is the same or different, preferably the same and represents -CH₂-O-(C₁-C₃-alkyl), -(CH₂)₂-O-CH₃, -(C₁-C₃-alkyl)-O-CH₃, -CH₂-S-(C₁-C₃-alkyl), -(CH₂)₂-S-CH₃, -(C₁-C₃-alkyl)-S-CH₃, -CH₂-O-CH₃, -CH₂-O-C₂H₅, -CH₂-O-C₂H₄OH, -CH₂-CH₂-O-CH₃, -CH₂-S-CH₃, -CH₂-S-C₂H₅, -CH₂-S-C₂H₄OH or -CH₂-CH₂-S-CH₃, most preferably -CH₂-CH₂-O-CH₃.

In a more preferred embodiment, M represents hydrogen or sodium and R1 is the same and represents -CH₂-CH₂-O-CH₃.

The trityl radicals of formula (I) may be synthesized as described in detail in WO-A-91/12024, WO-A-96/39367, WO 97/09633 and WO-A-98/39277. Briefly, the radicals may be synthesized by reacting three molar equivalents of a metallated monomeric aryl compound with one molar equivalent of a suitably protected carboxylic acid derivative to form a trimeric intermediate. This intermediate is metallated and subsequently reacted with e.g. carbon dioxide to result in a tri-carboxylic trityl carbinol which, in a further step, is treated with a strong acid to generate a triarylmethyl cation. This cation is then reduced to form the stable trityl radical.

A liquid mixture comprising ¹³C-pyruvate and/or ¹³C-pyruvic acid and optionally a solvent preferably contains 5 to 100 mM trityl radicals of formula (I), more preferably 10 to 20 mM, especially preferably 12 to 18 mM and most preferably 13 to 17 mM.

It has been found that the build-up time for polarisation in the DNP process is shorter using higher amounts of radical, however, the achievable polarisation level is lower. Hence these two effects have to be balanced against each other.

The DNP technique is for example described in WO-A-98/58272 and in WO-A-01/96895. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. In a preferred embodiment of the method of the invention, the DNP process is carried out in liquid helium and a magnetic field of about 1 T or above. Suitable polarisation units are for instance described in WO-A-02/37132. In a preferred embodiment, the polarisation unit comprises a cryostat and polarising means, e.g. a microwave chamber connected by a wave guide to a microwave source in a central bore surrounded by magnetic field producing means such as a superconducting magnet. The bore extends vertically down to at least the level of a region P near the superconducting magnet where the magnetic field strength is sufficiently high, e.g. between 1 and 25 T, for polarisation of the ¹³C nuclei to take place. The sample bore is preferably sealable and can be evacuated to low pressures, e.g. pressures in the order of 1 mbar or less. A sample (i.e. the mixture comprising the paramagnetic agent and ¹³C-pyruvate and/or ¹³C-pyruvic acid) introducing means such as a removable sample-transporting tube can be contained inside the bore and this tube can be inserted from the top of the bore down to a position inside the microwave chamber in region P. Region P is cooled by liquid helium to a temperature low enough to for polarisation to take place, preferably temperatures of the order of 0.1 to 100 K, more preferably 0.5 to 10 K, most preferably 1 to 5 K. The sample introducing means is preferably sealable at its upper end in any suitable way to retain the partial vacuum in the bore. A sample-retaining container, such as a sample-retaining cup, can be removably fitted inside the lower end of the sample introducing means. The sample-retaining container is preferably made of a light-weight material with a low specific heat capacity and good cryogenic properties such, e.g. KelF (polychlorotrifluoroethylene) or PEEK (polyetheretherketone). The sample container may hold one or more samples to be polarised.

The sample is inserted into the sample-retaining container, submerged in the liquid helium and irradiated with microwaves, preferably at a frequency about 94 GHz at 200 mW. The level of polarisation may be monitored by acquiring solid state ¹³C-NMR signals of the sample during microwave irradiation, thus the use of a polarising unit containing means to acquire solid state ¹³C-NMR spectra in step b) is preferred. Generally, a saturation curve is obtained in a graph showing ¹³C-NMR signal vs. time. Hence it is possible to determine when the optimal polarisation level is reached.

If hyperpolarisation is carried out by a method that requires the sample to be in the solid state, e.g. by the DNP method, the solid sample must be transferred into the liquid state to employ it in the method of the invention. The solid polarised mixture is either dissolved, like for instance described in WO-A-02/37132 or melted, as for instance described in WO-A-02/36005. Dissolution of the solid hyperpolarised sample is preferred, more preferred the dissolution in a buffer, preferably a physiologically tolerable buffer, to obtain a liquid composition. The term "buffer" in the context of this application denotes one or more buffers, i.e. also mixtures of buffers.

Preferred buffers are physiologically tolerable buffers, more preferably buffers which buffer in the range of about pH 7 to 8 like for instance phosphate buffer (KH₂PO₄/Na₂HPO₄), ACES, PIPES, imidazole/HCl, BES, MOPS, HEPES, TES, TRIS, HEPPS or TRICIN. More preferred buffers are phosphate buffer and TRIS, most preferred is TRIS. In another embodiment, more than one of the aforementioned preferred buffers, i.e. a mixture of buffers, is used.

When ¹³C-pyruvic acid was used as the compound to be polarised, the dissolution also encompasses the conversion of ¹³C-pyruvic acid to ¹³C-pyruvate. To achieve this, ¹³C-pyruvic acid is reacted with a base. In one embodiment, ¹³C-pyruvic acid is reacted with a base to convert it to ¹³C-pyruvate and subsequently a buffer is added. In another preferred embodiment the buffer and the base are combined in one solution and this solution is added to ¹³C-pyruvic acid, dissolving it and converting it into ¹³C-pyruvate at the same time. In a preferred embodiment, the base is an aqueous solution of NaOH, Na₂CO₃ or NaHCO₃, most preferred the base is NaOH. In a particularly preferred embodiment, a solution of TRIS buffer containing NaOH is used to dissolve ¹³C-pyruvic acid and convert it into the sodium salt of ¹³C-pyruvate.

In another preferred embodiment, the buffer or - where applicable - the combined buffer/base solution further comprises one or more compounds which are able to bind or complex free paramagnetic ions, e.g. chelating agents like DTPA or EDTA. It has been found that free paramagnetic ions may cause shortening of the T₁ of the hyperpolarised compound, which is preferably avoided.

The dissolution may be carried out by preferably using the methods and/or devices disclosed in WO-A-02/37132. If hyperpolarisation was carried out by the DNP method, a dissolution unit may be used which is either physically separated from the polariser or is a part of an apparatus that contain the polariser and the dissolution unit. In a preferred embodiment dissolution is carried out at an elevated magnetic field to improve the relaxation and retain a maximum of the hyperpolarisation. Field nodes should be avoided and low field may lead to enhanced relaxation despite the above measures.

If hyperpolarisation is carried out by the DNP method, the paramagnetic agent and/or reaction products thereof are preferably removed from the ¹³C-pyruvate containing solution. The paramagnetic agent and/or reaction products may be removed partially, substantially or ideally completely, the complete removal is preferred. Reaction products of for instance trityl radicals of the formula (I) might be esters which may be formed upon reaction of pyruvic acid with radicals of formula (I) comprising hydroxy groups. Methods usable to remove the paramagnetic agent and/or reaction products thereof are known in the art. Generally, the methods applicable depend on the nature of the paramagnetic agent and/or its reaction products. Upon dissolution of the solid sample after polarisation, the radical might precipitate and it may easily be separated from the liquid composition by filtration. If magnetic particles are used as paramagnetic agents, these particles are easily removed by filtration as well. If no precipitation occurs, the paramagnetic agent may be removed by chromatographic separation techniques, e.g. liquid phase chromatography like reversed phase, straight phase or ion exchange chromatography or by extraction.

As trityl radicals of formula (I) have a characteristic UV/visible absorption spectrum, it is possible to use UV/visible absorption measurement as a method to check for its existence in the liquid composition after its removal. In order to obtain quantitative results, i.e. the concentration of the radical present in the dissolved hyperpolarised sample, the optical spectrometer can be calibrated such that absorption at a specific wavelength form a sample yields the corresponding radical concentration in the sample.

The isotopic enrichment of the ¹³C-pyruvate used in the method of the invention - and/or the 13C-pyruvic acid which is preferably used to obtain hyperpolarised ¹³C-pyruvate by the DNP method, is preferably at least 75%, more preferably at least 80% and especially preferably at least 90%, an isotopic enrichment of over 90% being most preferred. Ideally, the enrichment is 100% ¹³C-pyruvic acid and/or ¹³C-pyruvate may be isotopically enriched at the C1-position (in the following denoted ¹³C₁-pyruvic acid and ¹³C₁-pyruvate), at the C2-position (in the following denoted ¹³C₂-pyruvic acid and ¹³C₂-pyruvate), at the C3-position (in the following denoted ¹³C₃-pyruvic acid and ¹³C₃-pyruvate), at the C1- and the C2-position (in the following denoted ¹³C_{1,2}-pyruvic acid and ¹³C_{1,2}-pyruvate), at the C1- and the C3-position (in the following denoted ¹³C₁,₃-pyruvic acid and ¹³C₁,₃-pyruvate), at the C2- and the C3-position (in the following denoted ¹³C₂,₃-pyruvic acid and ¹³C_{2,3}-pyruvate) or at the C1-, C2- and C3-position (in the following denoted ¹³C₁,_{2,3}-pyruvic acid and ¹³C₁,_{2,3}-pyruvate); the C1-position being the preferred one.

Several methods for the synthesis of ¹³C₁-pyruvic acid and ¹³C₁-pyruvate are known in the art. Briefly, Seebach et al., Journal of Organic Chemistry 40(2), 1975, 231-237 describe a synthetic route that relies on the protection and activation of a carbonyl-containing starting material as an S,S-acetal, e.g. 1,3-dithian or 2-methyl-1,3-dithian. The dithian is metallated and reacted with a methyl-containing compound and/or ¹³CO₂. By using the appropriate isotopically enriched ¹³C-component as outlined in this reference, it is possible to obtain ¹³C₁-pyruvate, ¹³C₂-pyruvate or ¹³C₁,₂-pyruvate. The carbonyl function is subsequently liberated by use of conventional methods described in the literature. A different synthetic route starts from acetic acid, which is first converted into acetyl bromide and then reacted with Cu¹³CN. The nitril obtained is converted into pyruvic acid via the amide (see for instance S.H. Anker et al., J. Biol. Chem. 176 (1948), 1333 or J. E. Thirkettle, Chem Commun. (1997), 1025). Further,¹³C-pyruvic acid may be obtained by protonating commercially available sodium ¹³C-pyruvate, e.g. by the method described in US patent 6,232,497.

To be used in the method of the invention, the hyperpolarised ¹³C-pyruvate is provided as a composition that is suitable for administration to a living human or non-human animal body. The composition preferably comprises a buffer or a mixture of buffers as described above. The composition may further comprise conventional pharmaceutically acceptable carriers, excipients and formulation aids. Thus, the composition may for example include stabilizers, osmolality adjusting agents, solubilizing agents and the like.

Pyruvate is an endogenous compound which is very well tolerated by the human body, even in high concentrations. As a precursor in the citric acid cycle, pyruvate plays an important metabolic role in the human body. Pyruvate is converted into different compounds: its transamination results in alanine, via oxidative decarboxylation, pyruvate is converted into acetyl-CoA and bicarbonate, the reduction of pyruvate results in lactate and its carboxylation in oxaloacetate.

It has now been found that the conversion of hyperpolarised ¹³C-pyruvate to hyperpolarised ¹³C-lactate, hyperpolarised ¹³C-bicarbonate (in the case of ¹³C₁-pyruvate, ¹³C_{1,2}-pyruvate or ¹³C_{1,2},₃-pyruvate only) and hyperpolarised ¹³C-alanine can be used for the discrimination between tumour tissue and healthy tissue using *in vivo* MR imaging. This is surprising as one has to bear in mind that the T₁ of hyperpolarised compounds decays due to relaxation and dilution. ¹³C₁-pyruvate has a T₁ relaxation in human full blood at 37 °C of about 42 s, however, the conversion of hyperpolarised ¹³C-pyruvate to hyperpolarised ¹³C-lactate, hyperpolarised ¹³C-bicarbonate and hyperpolarised ¹³C-alanine has been found to be fast enough to allow signal detection from the ¹³C-pyruvate parent compound and its metabolites. The amount of alanine, bicarbonate and lactate is dependent on the metabolic status of the tissue under investigation. The MR signal intensity of hyperpolarised ¹³C-lactate, hyperpolarised ¹³C-bicarbonate and hyperpolarised ¹³C-alanine is related to the amount of these compounds and the degree of polarisation left at the time of detection, hence by monitoring the conversion of hyperpolarised ¹³C-pyruvate to hyperpolarised ¹³C-lactate, hyperpolarised ¹³C-bicarbonate and hyperpolarised ¹³C-alanine it is possible to study metabolic processes *in vivo* in the human or non-human animal body by using non-invasive MR imaging.

It has been found that the MR signal amplitudes arising from the different pyruvate metabolites vary depending on the tissue type. The unique metabolic peak pattern formed by alanine, lactate, bicarbonate and pyruvate can be used as fingerprint for the metabolic state of the tissue under examination and thus allows for the discrimination between healthy tissue and tumour tissue. This makes a composition comprising hyperpolarised ¹³C-pyruvate an excellent agent for *in vivo* MR tumour imaging.

Generally, the subject under examination, e.g. patient or animal, is positioned in the MR magnet. Dedicated ¹³C-MR RF-coils are positioned to cover the area of interest.

The composition comprising ¹³C-pyruvate, is administered parenterally, preferably intravenously, intra-arterially or directly into the region or organ of interest. Dosage and concentration of the composition according to the invention will depend upon a range of factors such as toxicity, the organ targeting ability and the administration route. Generally, the composition is administered in a concentration of up to 1 mmol pyruvate per kg bodyweight, preferably 0.01 to 0.5 mmol/kg, more preferably 0.1 to 0.3 mmol/kg. The administration rate is preferably less than 10 ml/s, more preferably less than 6 ml/min and most preferable of from 5 ml/s to 0.1 ml/s. At less than 400s after the administration, preferably less than 120 s, more preferably less than 60 s after the administration, especially preferably 20 to 50 s after the administration and most preferably 30 to 40 s after the administration, an MR imaging sequence is applied that encodes the volume of interest in a combined frequency and spatial selective way. This will result in metabolic images of ¹³C-lactate, ¹³C-alanine and ¹³C-pyruvate and more preferably in metabolic images of ¹³C-lactate, ¹³C-alanine, ¹³C-bicarbonate and ¹³C-pyruvate. Within the same period of time, a proton image with for without a proton MRI contrast agent may be acquired to obtain anatomical and/or perfusion information.

The encoding of the volume of interest can be achieved by using so-called spectroscopic imaging sequences as described in for instance T.R. Brown et al., Proc. Natl. Acad. Sci. USA 79, 3523-3526 (1982); A.A. Maudsley, et al., J. Magn. Res 51, 147-152 (1983). Spectroscopic image data contain a number of volume elements in which each element contains a full ¹³C-MR spectrum. ¹³C-pyruvate and its ¹³C-metabolites all have their unique position in a ¹³C-MR spectrum and their resonance frequency can be used to identify them. The integral of the peak at its resonance frequency is directly linked to the amount of ¹³C-pyruvate and its ¹³C-metabolites, respectively. When the amount of ¹³C-pyruvate and each ¹³C-metabolite is estimated using for example time domain fitting routines as described for instance in L. Vanhamme et al., J Magn Reson 129, 35-43 (1997), images can be generated for ¹³C-pyruvate and each ¹³C-metabolite in which a colour coding or grey coding is representative for the amount of ¹³C-pyruvate and each ¹³C-metabolite measured.

Although spectroscopic imaging methods have proven their value in producing metabolic images using all kind of MR nuclei e.g.¹H, ³¹P, ²³Na, the amount of repetitions needed to fully encode the spectroscopic image makes this approach less suitable for hyperpolarised ¹³C. Care has to be taken to ensure hyperpolarised ¹³C- signal is available during the whole MR data acquisition. At the expense of a reduced signal to noise, this can be achieved by reducing the RF-pulse angle that is applied in every phase encoding step. Higher matrix sizes require more phase encoding steps and longer scan times.

Imaging methods based on the pioneering work by P. C. Lauterbur (Nature, 242, 190-191, (1973) and P. Mansfield (J. Phys. C. 6, L422-L426 (1973)), implying applying a readout gradient during the data acquisition, will allow for higher signal to noise images or the equivalent, higher spatial resolution images. However, these imaging methods in their basic form will not be able to produce separate images for ¹³C-pyruvate and its ¹³C-metabolites but an image containing the signals of ¹³C-pyruvate and all of its ¹³C-metabolites, i.e. the identification of specific metabolites is not possible.

In a preferred embodiment, imaging sequences are used that will make use of multiechoes to code for the frequency information. Sequences that can produce separate water and fat ¹H-images are for example described in G. Glover, J Magn Reson Imaging 1991;1:521-530 and S. B. Reeder et al., MRM 51 35-45 (2004). Since the metabolites to be detected and as such their MR frequencies are known, the approach discussed in the references above can be applied to acquire direct images of ¹³C-pyruvate, ¹³C-alanine and ¹³C-lactate and preferably ¹³C-pyruvate, ¹³C-alanine, ¹³C-lactate and ¹³C-bicarbonate. This procedure makes more efficient use of the hyperpolarised ¹³C-MR signal, giving a better signal quality compared to the classical spectroscopic imaging technique, a higher spatial resolution and faster acquisition times.

Tumour tissue is often characterised by an increased perfusion and higher metabolic activity. The process of increasing the vascular bed, angiogenesis, is induced by cells that due to their higher metabolic needs and/or their larger distance from a capillary are not able to get enough substrates that can provide the energy needed to sustain energy homeostasis. It is in this area, where cells have problems in producing enough energy a marked change in metabolic pattern is expected. Tissue with problems sustaining energy homeostasis will alter its energy metabolism which in particular results in an increased lactate production. Surprisingly, it is possible to make this change in metabolism visible using hyperpolarised ¹³C-pyruvate within the short MR imaging time window available, i.e. using the high ¹³C-lactate signal in the tumour area to discriminate the tumour from healthy tissue. As the perfusion is heterogeneous in tumour tissue, it is preferred to correct the ¹³C-lactate signal for the amount of pyruvate (¹³C-yruvate signal) available in the same region. By this correction, a weighted lactate over pyruvate image is obtained. This will allow for emphasising regions in the tissue with a relative high lactate signal with respect to the pyruvate signal and thus improve the discrimination between tumour tissue and healthy tissue.

To correct for the pyruvate signal, both lactate and pyruvate images are normalized to the maximum value in each individual image. Second, the normalized lactate image is multiplied by the inverted pyruvate image, e.g. the maximum pyruvate signal in the image minus the pyruvate level for every pixel. As a last step, the intermediate result gained in the operation above is multiplied by the original lactate image.

To emphasise regions with altered metabolism, the high ¹³C-lactate signal in connection with a reduced ¹³C-alanine signal can be used in a similar operation as described in the paragraph above, whereby a weighted lactate over alanine image is obtained. Surprisingly, the identification of the tumour area, i.e. the discrimination between tumour tissue and healthy tissue is improved by this correction as well. To correct for the alanine signal, both lactate and alanine images are normalized to the maximum value in each individual image. Second, the normalized lactate image is multiplied by the inverted alanine image, e.g. the maximum alanine signal in the image minus the alanine level for every pixel. As a last step, the intermediate result gained in the operation above is multiplied by the original lactate image. In a similar manner, the ¹³C-bicarbonate signal may be included in the analysis as well. Further a proton image acquired with our without a proton MRI contrast agent may be included in the analysis to obtain anatomical and/or perfusion information.

In another preferred embodiment, the composition comprising hyperpolarised ¹³C-pyruvate is administered repeatedly, thus allowing dynamic studies. This is a further advantage of the method according to the invention in comparison to other MR tumour imaging methods employing MR imaging agents which, due to their relatively long circulation in the patient's body, do not allow such dynamic studies.

The method according to the invention can further be used for *in vivo* MR tumour staging. The same metabolic images and/or metabolic weighted images as described in the preceding paragraphs may be used for this purpose with appropriate cut off categories defined dependent on tumour size and metabolic activity.

Further, the method according to the invention can be used for *in vivo* MR tumour therapy monitoring, e.g. by monitoring direct changes in metabolism pattern of tumours upon treatment with therapeutic antitumour agents and/or radiation treatment or in connection with any type of interventional techniques with or without any kind of ablation, i.e. chemical ablation combined with radio frequencies, microwaves or ultrasound.

Tumour MR imaging according to the method of the invention can be influenced and improved by preparing the patient or the animal in a way that will perturb the protein metabolism, lipid metabolism or energy metabolism in general. Ways to achieve this are known in the art, e.g. by abrosia (for instance over night), glucose infusion and the like.

In a preferred embodiment; the method according to the invention is used for *in vivo* MR tumour imaging, tumour therapy monitoring and/or tumour staging of brain tumours, breast tumours, colon tumours, lung tumours, kidney tumours, head and neck tumours, muscle tumours, ovarian tumours, gastric tumours, pancreatic tumours, esophageal tumours and prostate tumours. It has further been found that the method according to the invention is especially useful for *in vivo* MR prostate tumour imaging, i.e. prostate tumour diagnosis and/or prostate tumour staging and/or prostate tumour therapy monitoring.

When a man presents to the doctor with symptoms of urinary pain or discomfort, prostate cancer is suspected. If the man is over 50 years, a Prostate Specific Antigen (PSA) test is performed. Prostate cancer is suspected on the basis of an elevated PSA and/or abnormal Digital Rectal Examination (DRE). If the PSA test is positive, the patient is sent to a specialist (an urologist) for diagnosis using ultrasound guided biopsy. Of the two million biopsy procedures per year performed in the US and Europe, 5 out of 6 and 2 out of 3 are negative, respectively. When detected at an early stage, the five-year survival rate for these patients is 100%. As prostate cancer is the most common cancer and the second leading cause of cancer death in men, there is a strong medical demand for a method for the diagnosis of prostate tumours which is capable of detecting prostate tumours at an early stage and which could help to reduce the number of biopsy procedures. The ¹³C-imaging of the prostate requires a transmit-receive volume ¹³C-RF-coil, preferably, a transmit volume ¹³C-RF-coil in combination with a MR receive only endorectal RF-coil is used and more preferably, a transmit-receive phased array volume ¹³C-RF-coil in combination with a MR receive only endorectal ¹³C-RF-coil is used. Especially preferred are coils that make the acquisition of a¹H-prostate image possible after the ¹³C-imaging.

### Examples

### Example 1: Synthesis of Tris(8-carboxy-2,2,6,6-(tetra(methoxyethyl)benzo[1,2-4,5']bis-(1,3)dithiole -4-yl)methyl sodium salt

10 g (70 mmol) Tris(8-carboxy-2,2,6,6-(tetra(hydroxyethyl)benzo-[1,2-4,5']-bis-(1,3)-dithiole-4-yl)methyl sodium salt which had been synthesized according to Example 7 of WO-A1-98/39277 were suspended in 280 ml dimethylacetamide under an argon atmosphere. Sodium hydride (2.75 g) followed by methyl iodide (5.2 ml) was added and the reaction which is slightly exothermic was allowed to proceed for 1 hour in a 34°C water bath for 60 min. The addition of sodium hydride and methyl iodide was repeated twice with the same amounts of each of the compounds and after the final addition, the mixture was stirred at room temperature for 68 hours and then poured into 500 ml water. The pH was adjusted to pH > 13 using 40 ml of 1 M NaOH (aq) and the mixture was stirred at ambient temperature for 15 hours to hydrolyse the formed methyl esters. The mixture was then acidified using 50 ml 2 M HCl (aq) to a pH of about 2 and 3 times extracted the ethyl acetate (500 ml and 2 x 200 ml). The combined organic phase was dried over Na₂SO₄ and then evaporated to dryness. The crude product (24 g) was purified by preparative HPLC using acetonitrile/water as eluents. The collected fractions were evaporated to remove acetonitrile. The remaining water phase was extracted with ethyl acetate and the organic phase was dried over Na₂SO₄ then evaporated to dryness. Water (200 ml) was added to the residue and the pH was carefully adjusted with 0.1 M NaOH (aq) to 7, the residue slowly dissolving during this process. After neutralization, the aqueous solution was freeze dried.

### Example 2: Production of a composition comprising hyperpolarised ¹³C-pyruvate by the DNP method using ¹³C-pyruvic acid and the trityl radical of Example 1

A 20 mM solution was prepared by dissolving 5.0 mg of the radical of Example 1 in ¹³C₁-pyruvic acid (164 µl). The sample was mixed to homogeneity and an aliquot of the solution (41 mg) was placed in a sample cup and inserted in the DNP polariser.

The sample was polarised under DNP conditions at 1.2 K in a 3.35 T magnetic field under irradiation with microwave (93.950 GHz). After 2 hours the polarisation was stopped and the sample was dissolved using a dissolution device according to WO-A-02/37132 in an aqueous solution of sodium hydroxide and tris(hydroxymethyl)-aminomethane (TRIS) to provide a neutral solution of hyperpolarised sodium ¹³C₁-pyruvate. The dissolved sample was rapidly analysed with ¹³C-NMR to assess the polarisation and a 19.0 % ¹³C polarisation was obtained.

### Example 3: Production of a composition comprising hyperpolarised ¹³C-pyruvate by the DNP method using ¹³C-pyruvic acid and the trityl radical of Example 1

A 15 mM solution was prepared by dissolving the radical of Example 1 (209.1 mg) in a mixture of ¹³C₁-pyruvic acid (553 mg) and unlabelled pyruvic acid (10.505 g). The sample was mixed to homogeneity and an aliquot of the solution (2.015 g) was placed in a sample cup and inserted in the DNP polariser.

The sample was polarised under DNP conditions at 1.2 K in a 3.35 T magnetic field under irradiation with microwave (93.950 GHz). After 4 hours the polarisation was stopped and the sample was dissolved using a dissolution device according to WO-A-02/37132 in an aqueous solution of sodium hydroxide and tris(hydroxymethyl)aminomethane (TRIS) to provide a neutral solution of hyperpolarised sodium ¹³C₁-pyruvate with a total pyruvate concentration of 0.5 M in 100 mM TRIS buffer. In series with the dissolution device a chromatographic column was connected. The column consists of a cartridge (D = 38 mm; h = 10 mm) containing hydrophobic packing material (Bondesil-C18, 40UM Part #:12213012) supplied by Varian. The dissolved sample was forced through the column which selectively adsorbed the radical. The filtered solution was rapidly analysed with ¹³C-NMR to assess the polarisation, 16.5 % ¹³C polarisation was obtained. The residual radical concentration was subsequently analysed with a UV spectrophotometer at 469 nm and was determined to be below the detection limit of 0.1 µM.

### Example 4: Production of hyperpolarised ¹³C-pyruvate by the DNP method using¹³C-pyruvic acid and Tris(8-carboxy-2,2,6,6-tetra(hydroxyethoxy)methyl-benzo[1,2-d:4,5-d']bis(1,3)dithiole-4-yl)methyl sodium salt

Tris(8-carboxy-2,2,6,6-tetra(hydroxyethoxy)methyl-benzo[1,2-d:4,5-d']-bis-(1,3)-dithiole-4-yl)methyl sodium salt was synthesised as described in Example 29 in WO-A-97/09633.

A 20 mM solution was prepared by dissolving Tris(8-carboxy-2,2,6,6-tetra(hydroxyethoxy)methyl-benzo[1,2-d:4,5-d']-bis-(1,3)-dithiole-4-yl)methyl sodium salt in ¹³C₁-pyruvic acid (83.1 mg). The sample was mixed to homogeneity, placed in a sample cup and inserted in the DNP polariser. The sample was polarised under DNP conditions at 1.2 K in a 3.35 T magnetic field under irradiation with microwave (93.950 GHz). The ¹³C-NMR signal from the sample was acquired using a Varian Inova-200 NMR spectrometer. The DNP enhancement was calculated from a measurement of the thermal equilibrium ¹³C-NMR signal and the enhanced NMR signal. 16% ¹³C polarisation was obtained.

### Example 5: Tumour imaging using a composition comprising hyperpolarised ¹³C-pyruvate as imaging agent

### 5.1 Tumour animal model and tumour preparation

R3230AC is a rat mammary adenocarcinoma that can be maintained in female Fischer 344 rats. To establish the animal tumour model, a frozen vial of R32030 cells containing RPMI 1640, 10% FBS and 10% DMSO was rapidly thawed in 37 °C. Thereafter, the cell solution was transferred to FBS and increasing volumes of RPMI 1640 were added. Finally, the cell suspension was transferred to a 25 cm² growth flask and put into an incubator at 37 °C, 5% CO₂. Growth media were changed every other day. At the day of rat infection, removal of cells was carried out either by mechanical force or by means of trypsin. Cells were washed using phosphate buffer lacking calcium and magnesium. Trypsin (0.05% trypsin in 0.02% EDTA) was added for 2-5 min. Then, 5 ml FBS was added and the cells were transferred into a beaker containing RPMI 1640 with FCS and antibiotics (100 IU/ml penicillin, 100 IU/ml streptomycin and 2.5 µg/ml amphotericin B). The cell solution was centrifugated and the cell pellet was resuspended in 20 ml RPMI with FBS and antibiotics, centrifugation and resuspension was repeated. The cells were then aliquoted to vials containing 4 x 10⁶ cells/ml RPMI 1640. To obtain donor tumours, female Fischer 344 rats (Charles River, 180-200 g) were anaesthetised and 0.3 ml of the cell suspension was subcutaneously injected in the inguinal region on both sides. 15 and 22 days later, pieces of tumour were prepared as described in F.A. Burgener et al., Invest Radiol 22/6 (1987), 472-478; S. Saini et al., J. Magn. Reson. 129/1 (1997), 35-43). Two incisions were made on the ventral abdomen of recipient female Fischer rats. A tumour piece was inserted into each pocket and the incisions were closed. Rats were brought to imaging 12-14 days after tumour engrafting.

### 5.2 Rat preparation and proton MR imaging

Weighed rats were anaesthetised using isoflurane (2-3%) and kept on a heated table to ensure a body temperature of about 37 °C. A catheter was introduced into the tail vein and into the *arteria carotis communis sinistra*. The rats were transported to the MR machine and placed on a home-built pad that was heated to approx. 37 °C by means of circulating FC-104 Fluorinert. This liquid will not give rise to background signals in ¹H- and ¹³C-MR imaging. Anaesthesia was continued by means of 1-2% isoflurane delivered via a long tube to an open-breathing system at a rate of 0.4 Umin. The arterial catheter was connected via a T-tube to a pressure recorder and a pump delivering saline (rate 0.15 Umin) to prevent catheter clotting. Rats were positioned in a rat MR coil (Rapid Biomedical, Germany) and imaging using a standard proton MR imaging sequence to get anatomical information and to determine the location of the tumour.

### 5.3 ¹³C-MR imaging

Based on the proton frequency found by the MR system the MR frequency for ¹³C₁-alanine was calculated according to the following equation:
Frequency ¹³C₁-alanine = 0.25144 x [(system frequency proton x 1.00021) - 0.000397708]

The frequency calculated positioned the MR signal arising from ¹³C₁-alanine on resonance with ¹³C₁-lactate on the left and ¹³C₁-pyruvate resonating on the right of ¹³C₁-alanine. An unlocalised MR spectroscopy sequence was run to ensure that the 13C-MR coil and the system MR frequency had been set up correctly. The ¹³C-image location was positioned to cover the tumour (slice thickness 10 mm, in plane pixel size 5 x 5 mm²). In the reconstruction phase, the image data was zero-filled to result in 2.5 x 2.5 x 10 mm³ resolution. ¹³C₁-pyruvate in TRIS buffer (90 mM) was injected in a dose of 10 ml/kg during a period of 12 s with a minimum volume of 2 ml into the tail vein and 30 s after the start of the injection (i.e. 18 s after finishing the injection), the chemical shift ¹³C-MR sequence was started.

### 5.4 Analysis of the MR imaging data

MR imaging resulted in a matrix containing 16 x 16 elements in which each element or voxel/pixel contains a ¹³C-MR spectrum. In the reconstruction phase, the matrix was zero-filled to 32 x 32, a mathematical operation that helps to improve the spatial resolution. The dataset to be analysed contained 1024 spectra as was exported to Dicom^{®} format (DICOM is the registered trademark of the National Electrical Manufacturers Association for its standards publications relating to digital communications of medical information) for further analysis. About half of these spectra did not contain MR signals as the position of these voxels was outside the animal. A location within the animal revealed voxels with high pyruvate signals and negligible lactate and alanine signal (blood pool) while other voxels showed pyruvate, alanine and lactate in about equal intensity.

The amplitudes for pyruvate, alanine and lactate were estimated using time domain fitting procedures which included the following: the zero order phase is constant over the dataset, the first order phase is 1.4 ms, the line width or damping in the time domain is allowed to vary between 0.5 and 3 times the average line width of the whole dataset for each metabolite independently and the frequency is allowed to vary with 20 Hz in both directions with respect to the average frequency found over the whole dataset for the highest peak, which has to be identified by the user.

The amplitudes for lactate, alanine and pyruvate were reordered in a matrix and resampled to match the resolution of the proton anatomical MR image. The ¹³C-MR images were projected on the anatomical images using an automated procedure to obtain an operator-independent result. The results were displayed in image sets containing the anatomical proton image of the tumour in the rat, the metabolic ¹³C-image for pyruvate, lactate and alanine projected onto the anatomical image, images showing for every pixel
a) ([lactate]ₙₒᵣₘ x ([pyruvate]ₘₐₓ- [pyruvate])ₙₒᵣₘ) x [lactate] and
b) ([lactate]ₙₒᵣₘ x ([alanine]ₘₐₓ- [alanine])ₙₒᵣₘ) x [lactate]
in which the term "[....]norm represents the normalised amplitude, i.e. scaled to its highest value in the metabolic image and [lactate] the amplitude calculated.

A successful result for the discrimination of tumour tissue and healthy tissue in a metabolic ¹³C-MR image was defined as highest lactate signal in the tumour area or a high weighted ratio lactate over pyruvate in the tumour area and a high weighted lactate over alanine ratio in the same pixel location.

### 5.5 Biological analysis

Tumour sites were visually inspected to detect signs of bleeding. Tumours were liberated from the rat bodies, weighed and cut in half. Tumour interiors were inspected visually assessing homogeneity, necrosis and bleeding. The tumour tissues were stored in 4% formalin.

A tumour-bearing rat was considered to be appropriate for evaluation if the following criteria were met: tumour weight > 100 mg, no visible necrosis or cysts in the tumour interior, a body temperature above 35 °C and a mean arterial blood pressure above 60 mm Hg at time of MR investigation.

### 5.6 Results

In total 30 different tumours were imaged in 18 rats. 1 rat failed and 3 tumours failed the biological criteria described in the preceding paragraph 5.5. The remaining 26 tumours in 17 rats were homogenous and had a massive non-necrotic interior. The average polarisation of ¹³C₁-pyruvate at the time of injection was 21.2 ± 2.9% (mean ± SD) and the pH was 8.08 ± 0.14 (mean ± SD).

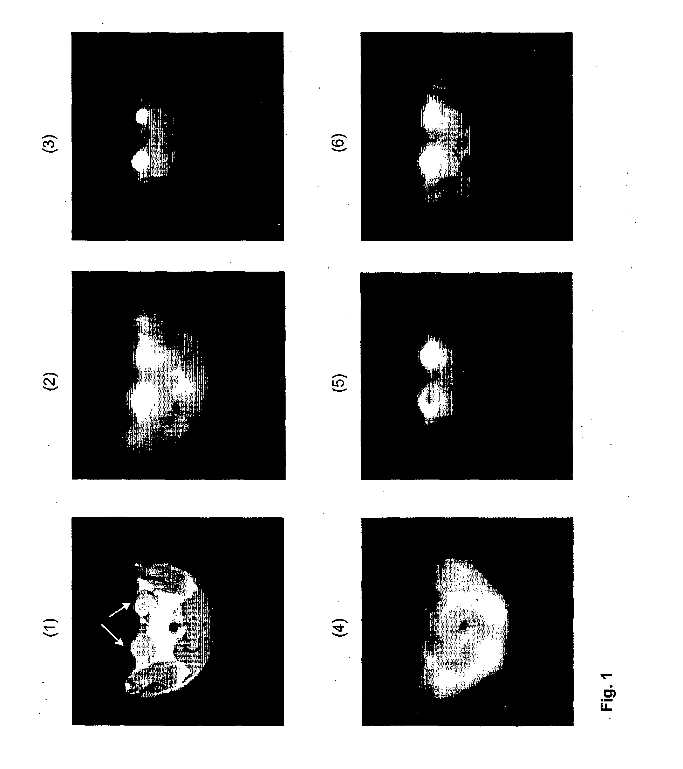

Figure 1 displays a typical set of images of one imaged rat with (1) the proton reference image, wherein the arrows indicate the tumour locations, (2) the ¹³C-pyruvate image, (3) the ¹³C-lactate image (4) the ¹³C-alanine image (5) the ¹³C-lactate image corrected for ¹³C-pyruvate and (6) the ¹³C-lactate image corrected for ¹³C-alanine. Images (2) to (6) are fused with the proton reference image.

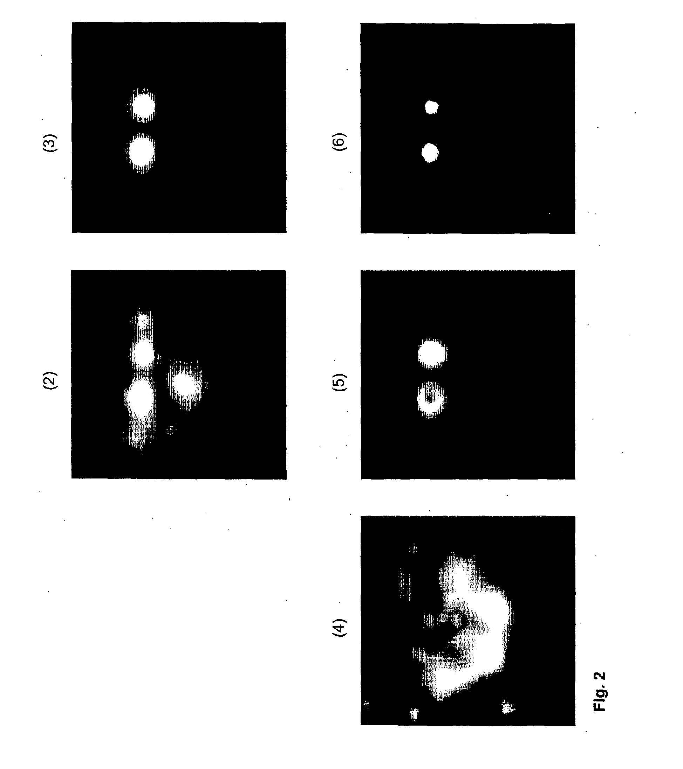

Figure 2 displays the same set of images, however with images (2) to (6) which are not fused with the anatomical proton image.

As a result, tumour location is indicated by a high pyruvate signal (2), due to high metabolic activity. However the lactate signal (3) ultimately identifies the correct location of the tumour. Alanine is visible in the skeletal muscle and is absent in the tumour tissue (4). The pyruvate and alanine corrected lactate images (5) and (6) result in an excellent contrast for the tumour as well.

It was thus demonstrated that the tumour location in the metabolic images is indicated by a high lactate signal, a high lactate signal corrected for pyruvate and a high lactate signal corrected for alanine.

The analysis of the metabolic ¹³C-MR images revealed a metabolic contrast in the tumour area in
- 24 out of 26 tumours for the lactate signal
- 26 out of 26 tumours for the lactate signal, pyruvate corrected (5.5,,a))
- 26 out of 26 tumours for the lactate signal, alanine corrected (5.5, b))

The overall rate of success for this study was 26 out of 26, or 100%.

With this study, it was demonstrated that tumours can be identified using hyperpolarised ¹³C₁-pyruvate which reaches the region of interest (tumour) in a time period which makes it possible to image the compound and its metabolites.

## Claims

1. Method for the discrimination between healthy and tumour tissue, said method comprising
(a) acquiring a ¹³C-MR image of ¹³C-pyruvate, a ¹³C-MR image of its ¹³C-containing metabolite alanine and a ¹³C-MR image of its ¹³C-containing metabolite lactate from a subject pre-administered with a composition comprising hyperpolarised ¹³C-pyruvate,
(b) correcting the lactate image for the amount of pyruvate and/or alanine by
multiplying the lactate image by the inverted pyruvate and/or alanine image, a high image signal within said corrected lactate image(s) being indicative of tumour tissue.

2. Method according to claim 1 wherein the hyperpolarised ¹³C-pyruvate is obtained by hyperpolarising ¹³C-pyruvic acid and/or ¹³C-pyruvate by the DNP method.

3. Method according to claim 1 or 2 wherein the composition comprising ¹³C-pyruvate further comprises one or more buffers selected from the group consisting of phosphate buffer (KH₂PO₄/Na₂HPO₄), ACES, PIPES, imidazole/HCl, BES, MOPS, HEPES, TES, TRIS, HEPPS and TRICIN.

4. Method according to claims 1 or 2 wherein imaging sequences that make use of multiechoes to code for frequency information are used for acquiring the ¹³C-images in step a).

5. Method according to claims 1 or 2 wherein the ¹³C-images in step a) are acquired at less than 400 s after the administration of the composition comprising ¹³C-pyruvate.

6. Method according to claims 1 or 2 wherein in addition a proton image with or without a proton MRI contrast agent is acquired.

7. Method according to claim 1 wherein said correction in step b) is carried out by
(i) normalizing the lactate and pyruvate and/or alanine images to the maximum value in each individual image
(ii) multiplying the normalized lactate image by the inverted pyruvate and/or alanine image; and
(iii) multiplying the results of step (ii) by the original lactate image.

8. Method according to claims 1 to 7 wherein the tumour is a brain tumour, breast tumour, colon tumour, lung tumour, kidney tumour, head and neck tumour, muscle tumour, ovarian tumour, gastric tumour, pancreatic tumour, esophageal tumour or prostate tumour.

9. Method according to claims 1 to 8 for *in vivo* MR tumour therapy monitoring and/or tumour staging.

## Patentansprüche

1. Verfahren zur Unterscheidung zwischen gesundem und Tumorgewebe, das Verfahren umfassend
(a) Aufnehmen eines ¹³C-MR-Bildes von ¹³C-Pyruvat, eines ¹³C-MR-Bildes von seinem ¹³C-haltigen Metaboliten Alanin und eines ¹³C-MR-Bildes von seinem ¹³C-haltigen Metaboliten Lactat, von einem Versuchslebewesen bzw. Patienten, dem zuvor eine hyperpolarisiertes ¹³C-pyruvat umfassende Zusammensetzung verabreicht worden ist,
(b) Korrigieren des Lactatbildes hinsichtlich der Menge von Pyruvat und / oder Alanin durch Multiplizieren des Lactatbildes mit dem invertierten Pyruvat- und / oder Alaninbild,
wobei ein hohes Bildsignal innerhalb des korrigierten Lactatbildes bzw. der korrigierten Lactatbilder ein Indikator für Tumorgewebe ist.

2. Verfahren nach Anspruch 1, wobei das hyperpolarisierte ¹³C-pyruvat erhalten wird durch Hyperpolarisieren von ¹³C-Brenztraubensäure und / oder ¹³C-Pyruvat mittels des DNP-Verfahrens.

3. Verfahren nach Anspruch 1 oder 2, wobei die ¹³C-pyruvat umfassende Zusammensetzung weiterhin einen oder mehrere Puffer umfasst, ausgewählt aus der Gruppe, die besteht aus Phosphatpuffer (KH₂P0₄/Na₂HPO₄), ACES, PIPES, Imidazol/HCl, BES, MOPS, HEPES, TES, TRIS, HEPPS und TRICIN.

4. Verfahren nach Anspruch 1 oder 2, wobei Bildgebungssequenzen, die Gebrauch machen von Multiechos, um für Frequenzinformationen zu codieren, zum Aufnehmen der ¹³C-Bilder in Schritt a) verwendet werden.

5. Verfahren nach Anspruch 1 oder 2, wobei die ¹³C-Bilder in Schritt a) weniger als 400 s nach der Verabreichung der ¹³C-pyruvat umfassenden Zusammensetzung aufgenommen werden.

6. Verfahren nach Anspruch 1 oder 2, wobei zusätzlich ein Protonenbild mit oder ohne Protonen-MRI-Kontrastmittel aufgenommen wird.

7. Verfahren nach Anspruch 1, wobei die Korrektur in Schritt b) ausgeführt wird durch
(i) Normieren der Lactat- und Pyruvat- und / oder Alaninbilder auf den Maximalwert in jedem einzelnen Bild,
(ii) Multiplizieren des normierten Lactatbildes mit dem invertierten Pyruvat-und/oder Alaninbild; und
(iii) Multiplizieren der Ergebnisse aus Schritt (ii) mit dem ursprünglichen Lactatbild.

8. Verfahren nach Ansprüchen 1 bis 7, wobei der Tumor ein Hirntumor, Mammatumor, Colontumor, Lungentumor, Nierentumor, Kopf-Hals-Tumor, Muskeltumor, Ovarialtumor, Magentumor, Pankreastumor, Ösophagustumor oder Prostatatumor ist.

9. Verfahren nach Ansprüchen 1 bis 8 zur *in vivo* MR-Tumortherapieüberwachung und / oder -Tumorstadienbestimmung.

## Revendications

1. Procédé de discrimination entre un tissu sain et tumoral, ledit procédé comprenant:
(a) l'acquisition d'une image par RM de ¹³C de pyruvate marqué par ¹³C, d'une image par RM de ¹³C de son métabolite alanine contenant du ¹³C et d'une image par RM de ¹³C de son métabolite lactate contenant du ¹³C à partir d'un sujet auquel a été préalablement administré une composition comprenant du pyruvate marqué par ¹³C hyperpolarisé,
(b) la correction de l'image de lactate en fonction de la quantité de pyruvate et/ou d'alanine en multipliant l'image de lactate par l'image de pyruvate et/ou d'alanine inversée, un signal d'image élevé à l'intérieur desdites images de lactate corrigées étant représentatif d'un tissu tumoral.

2. Procédé selon la revendication 1, dans lequel le pyruvate marqué par ¹³C hyperpolarisé est obtenu par hyperpolarisation d'acide pyruvique marqué par ¹³C et/ou de pyruvate marqué par ¹³C par le procédé DNP.

3. Procédé selon la revendication 1 ou 2, dans lequel la composition comprenant du pyruvate marqué par ¹³C comprend, en outre, un ou plusieurs tampons sélectionnés à partir du groupe constitué par un tampon de phosphate (KH₂PO₄/Na₂HPO₄), de l'ACES, du PIPES, de l'imidazole/HCl, du BES, du MOPS, de l'HEPES, du TES, du TRIS, de l'HEPPS et de la tricine.

4. Procédé selon les revendications 1 ou 2, dans lequel des séquences d'imagerie qui utilisent des échos multiples afin de coder des informations fréquentielles sont utilisées lors de l'acquisition des images sous ¹³C à l'étape a).

5. Procédé selon les revendications 1 ou 2, dans lequel les images sous ¹³C à l'étape a) sont acquises moins de 400 s après administration de la composition comprenant du pyruvate marqué par ¹³C.

6. Procédé selon les revendications 1 ou 2, dans lequel, en plus, une image protonique avec ou sans agent de contraste d'IRM protonique est acquise.

7. Procédé selon la revendication 1, dans lequel ladite correction à l'étape b) est mise en oeuvre par
(i) normalisation des images de lactate et de pyruvate et/ou d'alanine à la valeur maximum sur chaque image individuelle ;
(ii) multiplication de l'image de lactate normalisée par l'image de pyruvate et/ou d'alanine inversée ; et
(iii) multiplication des résultats de l'étape (ii) par l'image de lactate initiale.

8. Procédé selon les revendications 1 à 7, dans lequel la tumeur est une tumeur du cerveau, une tumeur du sein, une tumeur du colon, une tumeur des poumons, une tumeur rénale, une tumeur de la tête et du cou, une tumeur musculaire, une tumeur ovarienne, une tumeur gastrique, une tumeur pancréatique, une tumeur oesophagienne ou une tumeur de la prostate.

9. Procédé selon les revendications 1 à 8, afin d'assurer la surveillance de thérapie tumorale et/ou le suivi d'évolution tumorale par RM in vivo.
